# EUROPEAN PATENT APPLICATION

(11) **EP 4 286 390 A1**
(43) Date of publication of application: **06.12.2023**
(21) Application number: 22745295.0
(22) Date of filing: 27.01.2022
(51) Int. Cl.: C07D 495/04, A61K 31/407, A61P 35/00, A61P 29/00

(54) **HETEROCYCLIC AMIDE DERIVATIVE, PREPARATION METHOD THEREFOR, AND APPLICATION THEREOF**

(30) Priority: 28.01.2021 CN 202110118586
(71) Applicant: Shenzhen Zhongge Biological Technology Co., Ltd., Futian District Shenzhen, Guangdong 518017 (CN)
(72) Inventor: CHENG, Huimin, Shenzhen, Guangdong 518017 (CN); WEN, Xiaoming, Shenzhen, Guangdong 518017 (CN); ZHANG, Peiyu, Shenzhen, Guangdong 518017 (CN); LIU, Zhiqiang, Shenzhen, Guangdong 518017 (CN); LUO, Lan, Shenzhen, Guangdong 518017 (CN); LAI, Lipeng, Shenzhen, Guangdong 518017 (CN); MA, Jian, Shenzhen, Guangdong 518017 (CN); WEN, Shuhao, Shenzhen, Guangdong 518017 (CN)
(74) Representative: Kailuweit & Uhlemann Patentanwälte Partnerschaft mbB
(86) International application number: PCT/CN2022/074184
(87) International publication number: WO 2022/161422

(57) **Abstract**

The present disclosure relates to a heterocyclic amide derivative, a preparation method therefor and an application thereof. Specifically, the present disclosure relates to a compound of formula I, a preparation method therefor and an application thereof, a pharmaceutical composition containing the compound as an active ingredient, or a pharmaceutically acceptable salt thereof. The present disclosure further relates to a use of the compound of formula (I) in the treatment and prevention of EP₄-mediated diseases.

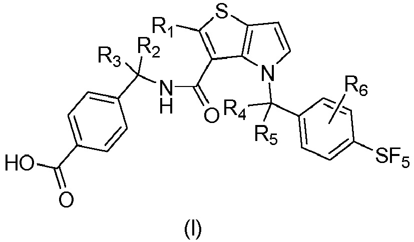

## Description

### TECHNICAL FIELD

The present invention belongs to the field of medicinal chemistry, in particularly relates to a heterocyclic amide derivative, the preparation method therefor and the application thereof.

### BACKGROUND

The characteristics and therapeutic applications of prostaglandin receptors and their most commonly used selective agonists and antagonists have been extensively studied. Prostaglandins are mediators that trigger pain, fever and other symptoms associated with inflammation. Prostaglandin E₂ (PGE₂) is the main metabolite of arachidonic acid associated with inflammation. In addition, prostaglandin E₂ is also involved in various physiological and/or pathological symptoms, such as hyperalgesia, uterine contractions, gastrointestinal peristalsis, arousal, inhibition of gastric acid secretion, blood pressure, platelet function, bone metabolism, angiogenesis and so forth. Four subtypes of prostaglandin E₂ receptors (EP₁, EP₂, EP₃ and EP₄) exhibit different pharmacological properties.

EP₄ receptors are characterized by having the longest intracellular C-terminal loop when compared to other prostaglandin receptors. EP₄ receptors are coupled to G proteins and mediate an increase in cyclic adenosine monophosphate concentration. The expression of EP₄ receptor is controlled by various physiological and pathophysiological processes, as this receptor is involved in ovulation and fertilization, induces bone formation, T cytokine signaling, prevents inflammatory bowel disease, promotes Langerhans cell migration and maturation, and mediates joint inflammation and other processes in collagen-induced arthritis models.

Studies have shown that elevated levels of cAMP mediated by EP₄ receptors are the main signal leading to immunosuppression in immune cells. In the context of APCmin mutations, knockout of EP₄ in mice compared with wild animals showed delayed tumorigenesis, indicating the protumor activity of PGE₂-EP₄ signaling in host immune cells.

Therefore, the compound disclosed in the present invention is expected to have a therapeutic effect on the treatment of EP₄ receptor-mediated diseases or conditions in mammals including humans, including acute and chronic pain, osteoarthritis, rheumatoid arthritis and cancer.

In conclusion, there is still an urgent need for novel compounds that can effectively and reliably inhibit EP₄ *in vitro* and *in vivo.*

### SUMMARY

The purpose of the present invention is to provide a new class of novel compound and a pharmaceutically acceptable salt thereof, with EP₄ receptor inhibitory activity and/or good pharmacodynamic/pharmacokinetic properties that can act as EP₄ receptor antagonists, and a pharmaceutical composition containing the same as an active ingredient, and a use of the same in the treatment or alleviation of EP₄ receptor-related diseases such as prostaglandin EP₄ receptor-mediated diseases.

In a first aspect, the present invention provides a compound of formula I or a pharmaceutically acceptable salt, hydrate, solvate, stereoisomer, isotope compound or prodrug thereof; 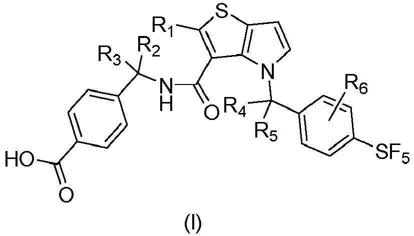 wherein,
R₁ is independently selected from the group consisting of: H, halogen, substituted or unsubstituted C₁₋₆ alkyl, and substituted or unsubstituted C₃₋₆ cycloalkyl;
R₂ and R₃ each is independently selected from the group consisting of: H, substituted or unsubstituted C₁₋₆ alkyl, and substituted or unsubstituted C₃₋₆ cycloalkyl; or, R₂ and R₃, together with the C atom to which they are attached, form a C₃₋₆ carbocyclic ring or a 3- to 6-membered heterocyclic ring containing one or two ring members each independently selected from the group consisting of S, O or NR_{b}; and the C₃₋₆ carbocyclic ring or 3- to 6-membered heterocyclic ring is optionally substituted with one or more Ri;
R_{b} each is independently selected from the group consisting of: H, substituted or unsubstituted C₁₋₆ alkyl, substituted or unsubstituted C₃₋₆ cycloalkyl, substituted or unsubstituted C₆₋₁₀ aryl, substituted or unsubstituted 5- to 6-membered heteroaryl, -C(O)R_{c}, and -S(O)₂R_{c};
R_{c} each is independently selected from the group consisting of: substituted or unsubstituted C₁₋₆ alkyl, substituted or unsubstituted C₃₋₆ carbocyclic ring, substituted or unsubstituted 3- to 6-membered heterocyclic ring, substituted or unsubstituted C₆₋₁₀ aryl, and substituted or unsubstituted 5- to 10-membered heteroaryl;
R₄ and R₅ each is independently selected from the group consisting of: H, and substituted or unsubstituted C₁₋₃ alkyl; or, R₄ and R₅, together with the C atom to which they are attached, form a substituted or unsubstituted C₃₋₆ carbocyclic ring (such as cyclopropyl); and
R₆ represents none or is selected from the group consisting of: halogen, CN, and haloalkyl (such as trifluoromethyl);
unless otherwise specified, the substitution refers to that one or more (such as 1, 2, or 3) of the hydrogens in a group is substituted by a substituent selected from the group consisting of: halogen (such as F), C₁₋₄ alkyl, and C₁₋₄ haloalkyl.

In a preferable embodiment, the substituted or unsubstituted C₁₋₆ alkyl is C₁₋₆ alkyl or C₁₋₆ haloalkyl; preferably, is C₁₋₆ alkyl or C₁₋₆ fluoroalkyl.

In a preferable embodiment, the substituted or unsubstituted C₃₋₆ cycloalkyl is C₃₋₆ cycloalkyl or C₃₋₆ halocycloalkyl; preferably, is C₃₋₆ alkyl or C₃₋₆ fluorocycloalkyl.

In a preferable embodiment, R₁ is independently selected from the group consisting of: H, halogen, C₁₋₆ alkyl, C₃₋₆ cycloalkyl, C₃₋₆ fluorocycloalkyl, C₁₋₆ fluoroalkyl;
R₂ and R₃ each is independently selected from the group consisting of: H, C₁₋₆ alkyl, C₃₋₆ cycloalkyl, C₃₋₆ fluorocycloalkyl, C₁₋₆ fluoroalkyl; or, R₂ and R₃, together with the C atom to which they are attached, form a C₃₋₆ carbocyclic ring or a 3- to 6-membered heterocyclic ring; wherein, the heterocyclic ring contains one or two ring members each independently selected from the group consisting of S, O or NR_{b}; and the C₃₋₆ carbocyclic ring or 3- to 6-membered heterocyclic ring is further optionally substituted with Ri;
R_{b} is selected from the group consisting of: H, C₁₋₆ alkyl, C₃₋₆ cycloalkyl, C₃₋₆ fluorocycloalkyl, C₁₋₆ fluoroalkyl, C₆₋₁₀ aryl, 5- to 10-membered heteroaryl, C(O)-C₁₋₆ alkyl, C(O)-C₆₋₁₀ aryl, S(O)₂-C₁₋₆ alkyl, and S(O)₂-C₆₋₁₀ aryl;
R₄ and R₅ each is independently H or C₁₋₃ alkyl; and
R₆ represents none or a substituent selected from the group consisting of: halogen, CN, and trifluoromethyl.

In a preferable embodiment, R₁ is selected from the group consisting of: H, halogen, C₁₋₆ alkyl, and C₃₋₆ cycloalkyl.

In a preferable embodiment, R₂ and R₃ each is independently selected from the group consisting of H, and C₁₋₆ alkyl, or, R₂ and R₃, together with the C atom to which they are attached, form cyclopropyl.

In a preferable embodiment, R₄ and R₅ each is independently H or methyl.

In a preferable embodiment, when R₆ is not none, R₆ is located in the ortho-position or meta-position of -SF₅.

In a preferable embodiment, R₆ represents none or is a substituent selected from the group consisting of: halogen.

In a preferable embodiment, R₁, R₂, R₃, R₄, R₅, R₆, R_{b} and R_{c} each is independently the corresponding group of the compounds in the Examples (such as the compounds listed in Table 1).

In a preferable embodiment, the compound of formula I or a pharmaceutically acceptable salt, hydrate, solvate, stereoisomer, isotope compound or prodrug thereof is a compound selected from the group consisting of: 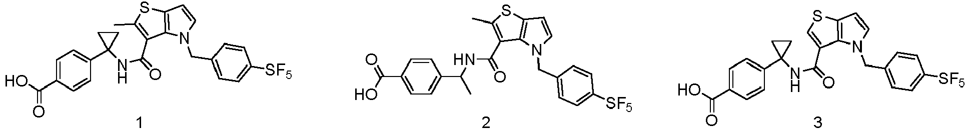 and

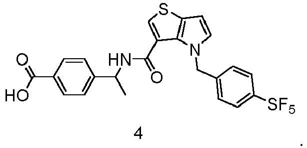

In a second aspect, the present invention provides a pharmaceutical composition, comprising the compound of formula I or a pharmaceutically acceptable salt, hydrate, solvate, stereoisomer, isotope compound or prodrug thereof according to the first aspect, and a pharmaceutically acceptable carrier or diluent.

In a third aspect, the present invention provides a use of the compound of formula I or a pharmaceutically acceptable salt, hydrate, solvate, stereoisomer, isotope compound or prodrug thereof according to the first aspect or the pharmaceutical composition according to the second aspect in the preparation of (i) a medicament for inhibiting the activity of EP₄ receptor and/or (ii) a medicament for the treatment or prevention of a disease associated with EP₄ receptor and/or (iii) a EP₄ receptor antagonist.

In a preferable embodiment, the disease associated with EP₄ receptor comprises a disease mediated by prostaglandin E₂ and/or prostaglandin EP₄ receptor.

In a preferable embodiment, the disease associated with EP₄ receptor comprises: acute and chronic pain, osteoarthritis, rheumatoid arthritis, cancer, or a combination thereof.

In a fourth aspect, the present invention provides a method for the treatment or prevention of a disease associated with EP₄ receptor, wherein the method comprises administering a therapeutically effective amount of the compound of formula I or a pharmaceutically acceptable salt, hydrate, solvate, stereoisomer, isotope compound or prodrug thereof according to the first aspect or the pharmaceutical composition according to the second aspect, to a subject in need thereof.

In a preferable embodiment, the subject in need thereof is a subject who has been identified or diagnosed with a EP₄ receptor-related disease.

In a fifth aspect, the present invention provides a method for inhibiting the activity of a EP₄ receptor in a cell or a subject, wherein the method comprises the step of contacting the cell with or administering to the subject a therapeutically effective amount of the compound of formula I or a pharmaceutically acceptable salt, hydrate, solvate, stereoisomer, isotope compound or prodrug thereof according to the first aspect or the pharmaceutical composition according to the second aspect.

In a preferable embodiment, the cell is a mammalian cell.

In a preferable embodiment, when the method is used to inhibit a cell, the method is an *in-vitro* non-therapeutic method.

In a preferable embodiment, the subject is a mammal, preferably human.

Within the scope of the present invention, the above technical features of the present invention and the technical features specifically described below (such as Examples) can be combined with each other, thus constituting a new or preferred technical solution. Due to space constraints, they are not repeated herein.

### DETAILED DESCRIPTION OF THE INVENTION

After extensive and in-depth research, the present inventors surprisingly discovered a class of compounds with novel structures with a good inhibitory activity toward a EP₄ receptor. In addition, the inventors also found that these compounds with novel structures had good pharmacodynamic/pharmacokinetic properties. On this basis, the present invention is completed.

### Definitions

In the present invention, unless otherwise specified, the terms used herein have general meanings well known to those skilled in the art.

When a substituent is described by a conventional chemical formula written from left to right, the substituent also includes a chemically equivalent substituent obtained when the structural formula is written from right to left. For example, -CH₂O- is equivalent to -OCH₂-.

As used herein, the term "alkyl" by itself or as part of another substituent, refers to a liner or branched hydrocarbon group with a specified number of carbon atoms (i.e., C₁₋₆ refers to one to six carbon atoms). Preferably, an alkyl generally contains 1-6 carbon atoms, i.e., C₁₋₆ alkyl. Examples of alkyl include, but not limited to, methyl, ethyl, n-propyl, isopropyl, n-butyl, tert-butyl, isobutyl, sec-butyl, n-amyl, n-hexyl and analogues thereof. One or more (such as 1-4) positions in an alkyl can be optionally substituted, and the substitution can be performed at any position in the group.

As used herein, the term "haloalkyl" refers to a branched or linear saturated aliphatic hydrocarbon group with a specified number of carbon atoms and substituted with one or more halogens. Examples of haloalkyl include, but not limited to, fluoromethyl, difluoromethyl, trifluoromethyl, trichloromethyl, pentafluoroethyl, pentachloroethyl, 2,2,2-trifluoroethyl, heptafluoropropyl and heptachloropropyl. Examples of haloalkyl further include "fluoroalkyl" in the form of a branched or linear saturated aliphatic hydrocarbon group with a specified number of carbon atoms and substituted with one or more fluorine atoms.

The term "fluoroalkyl" or "fluorinated alkyl" refers to the alkyl as defined above, in which one or more hydrogen atoms are substituted with a fluorine atom.

The term "alkoxy" refers to a linear or branched or cyclic alkyl connected by an ether oxygen, from which the free valence bond derives. Representative examples include (but not limited to): methoxy, ethoxy, propoxy, isopropoxy, and butoxy etc. C₁₋₃ alkoxy is preferred.

The term "fluoroalkoxy" or "fluoro-alkoxy" refers to the alkoxy as defined above, in which one or more hydrogen atoms are substituted with a fluorine atom.

The term "haloalkoxy" refers to -O- haloalkyl, including linear or branched or cyclic haloalkoxy. Representative examples include (but not limited to): fluoromethoxy, difluoromethoxy, trifluoromethoxy, trichloromethoxy, pentafluoroethoxy, and pentachloroethoxy.

The term "cycloalkyl" or "carbocyclic ring" refers to a cyclic alkyl including a saturated mono-cycle, bi-cycle or multi-cycle, such as C₃₋₈ or C₃₋₁₂ cycloalkyl. C₃₋₈ cycloalkyl refers to C₃, C₄, C₅, C₆, C₇, or C₈ cycloalkyl. Cycloalkyl may also include a cycloalkyl with a spiro ring, a bridge ring, or a fused ring. Representative cycloalkyl of the present invention includes, but not limited to: cyclopropyl, cyclobutyl, cyclopentyl, cyclohexyl and norcamphanyl. It should be understood that the substituted or unsubstituted cycloalkyl, for example branched cycloalkyl (such as 1-methylcyclopropyl and 2-methylcyclopropyl), is included in the definition of "cycloalkyl". C₅₋₁₂ fused bicyclic rings refer to C₅, C₆, C₇, C₈, C₉, C₁₀, C₁₁, and C₁₂ bicycloalkyl, which include but not limited to: 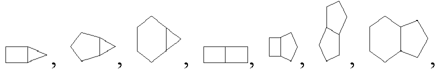 and 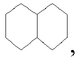 etc. C₅₋₁₂ spirobicyclic rings refer to C₅, C₆, C₇, C₈, C₉, C₁₀, C₁₁, and C₁₂ bicycloalkyl, which include but not limited to: 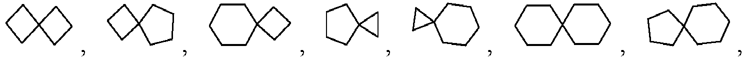 and 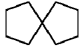 etc. In the present invention, cycloalkyl is preferably a monocyclic cycloalkyl containing 3 to 6 carbon atoms (i.e., C₃₋₆), such as cyclopropyl, cyclobutyl, cyclopentyl, or cyclohexyl.

The term "aryl" or "aromatic group", alone or as part of a group such as "aralkyl", "aryl alkoxy" or "aryl oxy alkyl", refers to a monocyclic, bicyclic or tricyclic ring system having a total of 5 to 15 ring members (or ring atoms) (preferably a 6- to 10-membered aromatic ring), wherein at least one ring in the system is aromatic and each ring in the system contains 3 to 7 ring members. "Aryl" can be substituted or unsubstituted. In some embodiments of the present invention, "aryl" refers to an aromatic ring system, which includes, but not limited to, phenyl, biphenyl, indanyl, 1-naphthyl, 2-naphthyl and tetrahydronaphthyl. Fused aryl can be connected to another group at a suitable location in a cycloalkyl ring or aromatic ring. The connection line drawn from within a ring system indicates that the bond can be connected to any suitable atom of the ring.

The term "heteroaryl" or "aromatic heterocyclic group" refers to a heteroaromatic system containing 1-4 heteroatoms and 5-14 ring atoms, wherein the heteroatom is selected from the group consisting of oxygen, nitrogen and sulfur. Heteroaryl is preferably a 5- to 10-membered ring, more preferably a 5- or 6-membered ring, such as pyrrolyl, pyrazolyl, imidazolyl, oxazolyl, isoxazolyl, thiazolyl, thiadiazolyl, isothiazolyl, furanyl, pyridinyl, pyrazinyl, pyrimidinyl, pyridazinyl, triazinyl, triazolyl and tetrazolyl. "Heteroaryl" can be substituted or unsubstituted, and when substituted, the substituents are preferably one or more groups, each of which is independently selected from the group consisting of alkyl, deuterated alkyl, haloalkyl, alkoxy, haloalkoxy, alkenyl, alkynyl, alkylthio, alkylamino, halogen, amino, nitro, hydroxyl, sulfhydryl, cyano, cycloalkyl, heterocyclic, aryl, heteroaryl, cycloalkylthio, oxo, carboxyl and carboxylate group.

The term "heterocycloalkyl" or "heterocyclic ring" refers to cycloalkyl containing one to five heteroatoms selected from the group consisting of N, O and S, wherein nitrogen and sulfur atoms are optionally oxidized and nitrogen atom is optionally quaternated. Heterocycloalkyl can be a monocyclic or bicyclic or multicyclic system. Non-limiting examples of heterocycloalkyl include pyrrolidine, imidazolidine, pyrazolidine, butyrolactam, valerolactam imidazolidinone, hydantoin, dioxolame, phthalimide, piperidine, 1,4-dioxane, morpholine, thiomorpholine, thiomorpholine-S-oxide, thiomorpholine-S,S-oxide, piperazine, pyran, pyridone, 3-pyrroline, thiapyran, pyranone, tetrahydrofuran, tetrahydrothiophene, quinine ring, etc. Heterocycloalkyl can be linked to the rest of the molecule through a ring carbon atom or a heteroatom.

The term "hydroxyl" refers to -OH.

The term "nitro" refers to -NO₂.

The term "amino" refers to -NH₂.

The term "halo" or "halogen" includes fluorine, chlorine, bromine and iodine.

The term "cyano" refers to -CN.

In the present invention, the above alkyl, haloalkyl, aryl, heteroaryl, alkenyl, alkynyl, etc. can be substituted or unsubstituted.

In the present invention, the term "substitution" refers to one or more hydrogen atoms on a particular group being substituted with a specific substituent. The specific substituents are the substituents described accordingly above, or the substituents that appear in each Example. Unless otherwise specified, a substituted group may have a substituent selected from a particular group at any substitutable site of the group, and the substituent can be the same or different in various positions. Those skilled in the art should understand that the combinations of substituents contemplated by the present invention are those that are stable or chemically achievable. Typical substitutions include, but not limited to, one or more of the following groups: such as, H, deuterium, halogen (such as single halogen substituents or polyhalogen substituents, the latter such as trifluoromethyl or alkyl containing Cl₃), nitrile, nitro, oxo (such as =O), trifluoromethyl, trifluoromethoxy, cycloalkyl, alkenyl, alkynyl, heterocyclic ring, aromatic ring, ORₐ, SRₐ, S(=O)Rₑ, S(=O)₂Rₑ, P(=O)₂Rₑ, S(=O)₂ORₑ, P(=O)₂ORₑ, NR_{b}R_{c}, NR_{b}S(=O)₂Rₑ, NR_{b}P(=O)₂Rₑ, S(=O)₂NR_{b}R_{c}, P(=O)₂NR_{b}R_{c}, C(=O)OR_{d}, C(=O)Rₐ, C(=O)NR_{b}R_{c}, OC(=O)Rₐ, OC(=O)NR_{b}R_{c}, NR_{b}C(=O)ORₑ, NR_{d}C(=O)NR_{b}R_{c}, NR_{d}S(=O)₂NR_{b}R_{c}, NR_{d}P(=O)₂NR_{b}R_{c}, NR_{b}C(=O)Rₐ, or NR_{b}P(=O)₂Rₑ, wherein, Rₐ can independently represent H, deuterium, alkyl, cycloalkyl, alkenyl, alkynyl, heterocyclic ring or aromatic ring, R_{b}, R_{c} and R_{d} can independently represent H, deuterium, alkyl, cycloalkyl, heterocyclic ring or aromatic ring, or R_{b} and R_{c}, together with the N atom, can form a heterocyclic ring; Rₑ can independently represent H, alkyl, cycloalkyl, alkenyl, alkynyl, heterocyclic ring or aromatic ring. The above typical substituents, such as alkyl, cycloalkyl, alkenyl, cycloalkenyl, alkynyl, heterocyclic ring or aromatic ring can be optionally substituted. The substituents are such as (but not limited to): halogen, hydroxyl, CN, carboxyl (-COOH), C₁₋₆ alkyl, C₂₋₆ alkenyl, C₂₋₆ alkynyl, C₃₋₈ cycloalkyl, 3- to 12-membered heterocyclic group, aryl, heteroaryl, C₁₋₈ aldehyde group, C₂₋₁₀ acyl, C₂₋₁₀ ester group, amido, C₁₋₆ alkoxy, C₁₋₁₀ sulfonyl, and C₁₋₆ ureido, etc.

Unless otherwise specified, it is assumed that any heteroatom that is not satisfied with the valence state has enough hydrogen atoms to supplement its valence state.

When the substituent is a non-terminal substituent, it is a subunit of the corresponding group, for example, alkyl corresponds to alkylene, cycloalkyl corresponds to cycloalkylene, heterocyclic group corresponds to heterocyclylene, alkoxy corresponds to oxyalkylene, etc.

The term "EP₄ antagonist" refers to a compound that inhibits or blocks cell signaling triggered by the interaction of PGE₂ with a EP₄ receptor, including but not limited to a compound of formula (I) described herein.

The term "treatment" refers to mitigating, inhibiting and/or reversing cancer progression in a subject in need thereof. The term "treatment" includes any indicator of successful treatment or improvement of cancer, including any objective or subjective parameters, such as reduction; remission; alleviating symptoms or making subjects more tolerant to an injury, pathology or condition; delaying or slowing down progression of disease, etc. Measurements of treatment or improvement can be based, for example, on the results of physical examinations, pathological examinations and/or diagnostic examinations known in the art. Treatment can also refer to reducing the occurrence or onset of cancer, or its recurrence (such as prolonged remission duration) compared with what may happen when no measures were taken.

The term "cancer" can include cancer caused by genetic mutations. Examples of such cancers include, but not limited to, breast cancer, cancers associated with Lee-Fraumeni syndrome such as childhood sarcoma, leukemia and brain cancer, cancers associated with Lynch syndrome, such as colon cancer, cholangiocarcinoma, brain cancer, endometrial cancer, kidney cancer, ovarian cancer, pancreatic cancer, small intestine cancer, stomach cancer and ureteral cancer, lung cancer, melanoma, prostate cancer, retinoblastoma, thyroid cancer and uterine cancer. Furthermore, cancer can be caused by acquired mutations, for example, diet, environment and/or lifestyle caused mutations or somatic mutations. Examples of such cancers may include, but not limited to, adrenal carcinoma, adrenal cortex carcinoma, bladder cancer, brain cancer, primary brain cancer, glioma, glioblastoma, breast cancer, cervical cancer, colon cancer (non-limiting examples include colorectal cancer such as colon adenocarcinoma and colon cancer), endometrial cancer, epidermal cancer, esophageal cancer, gallbladder cancer, genitourinary tract cancer, head and neck cancer, kidney cancer, liver cancer, lung cancer (non-limiting examples include adenocarcinoma, small cell lung cancer and non-small cell lung cancer), lymphoma (non-limiting examples include B-cell lymphoma, T-cell lymphoma, Hodgkin lymphoma, non-Hodgkin lymphoma), melanoma, malignant melanoma, malignant carcinoid, malignant pancreatic insulinoma, myeloma, multiple myeloma, ovarian cancer, pancreatic cancer (such as exocrine pancreatic cancer), prostate cancer, renal cell carcinoma, skin cancer, e.g., in addition to those mentioned above, squamous cell carcinoma, gastric cancer, testicular cancer, thyroid cancer, thyroid follicular carcinoma, Wilms' tumor, choriocarcinoma, mycosis, malignant hypercalcemia, cervical hyperplasia, leukemia, acute lymphoblastic leukemia, chronic lymphoblastic leukemia, hairy cell lymphoma, Burke's lymphoma, acute myelogenous leukemia, chronic myelogenous leukemia, myelodysplastic syndrome, promyelocytic leukemia, chronic myeloid leukemia, acute myeloid leukemia, fibrosarcoma, habdomyosarcoma, astrocytoma, neuroblastoma, rhabdomyosarcoma, schwannomas, Kaposi sarcoma, polycythemia, essential thrombocytosis, Hodgkin's disease, non-Hodgkin lymphoma, soft tissue sarcoma, osteogenic sarcoma, primary macroglobulinemia, seminoma, teratoma, osteosarcoma, xenodermatoma, keratokeratoma and retinoblastoma.

### Active ingredient

As used herein, the terms "compound according to the present invention" and "active ingredient according to the present invention" are used interchangeably to refer to a compound of formula I, or a pharmaceutically acceptable salt, hydrate, solvate, stereoisomer, isotope compound (such as deuterated compound) or prodrug thereof. The term also includes racemates and optical isomers.

The compound according to the present invention has the structure shown in formula I: 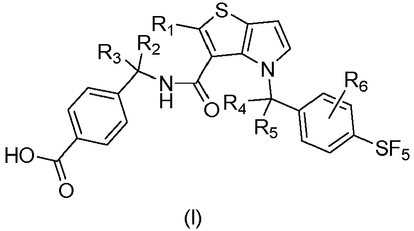 wherein, R₁, R₂, R₃, R₄, R₅, R₆, R_{b} and R_{c} are defined as above.

In the present invention, the salts that a compound may form also fall within the scope of the present invention. Unless otherwise specified, the compound according to the present invention should be understood to include its salts. The term "salt" as used herein, refers to salts formed with inorganic or organic acid or base to form an acidic salt or a basic salt. In addition, when the compound according to the present invention contains a basic fragment, it includes but not limited to pyridine or imidazole, and when the compound contains an acidic fragment, it includes but not limited to carboxylic acid. Zwitterions ("inner salts") that may be formed are contained within the scope of the term "salt". Pharmaceutically acceptable (i.e., non-toxic, physiologically acceptable) salts are preferred, although other salts are also useful, for example they can be used in isolation or purification steps during preparation of a compound. The compound according to the present invention may form salts. For example, compound I can be reacted with an amount (such as an equivalent amount) of acid or base, then salted out in a medium, or be obtained by freeze-drying in an aqueous solution.

The compound of the present invention may contain a basic fragment or moiety, including but not limited to amine or pyridine or imidazole ring, which may form a salt with an organic or inorganic acid. Typical acids that can be used to form a salt include acetate (such as acetic acid or trihaloacetic acid, such as trifluoroacetic acid), adipate, alginate, ascorbate, aspartate, benzoate, benzenesulfonate, bisulfate, borate, butyrate, citrate, camphor salt, camphorsulfonate, cyclopentane propionate, diethylene glycolate, dodecanyl alkyl sulfate, ethane sulfonate, fumarate, glucoheptanoate, glycerophosphate, hemisulfate, enanthate, hexanoate, hydrochloride, hydrobromide, hydroiodate, hydroxyethanesulfonate (such as 2-hydroxyethanesulfonate), lactate, maleate, mesylate, naphthalene sulfonate (such as 2-naphthalenesulfonate), nicotinate, nitrate, oxalate, pectinate, persulfate, phenpropionate (such as 3-phenpropionate), phosphate, picrate, neopentanoate, propionate, salicylate, succinate, sulfate (such as formed with sulfuric acid), sulfonate, tartrate, thiocyanate, tosylate such as p-toluenesulfonate, dodecanoate and so on.

Some compounds according to the present invention may contain an acidic fragment or moiety, including but not limited to carboxylic acid, which may form salts with various kinds of organic or inorganic bases. Typical salts formed from a base include ammonium salts, alkali metal salts such as sodium, lithium, and potassium salts, alkaline earth metal salts such as calcium, magnesium salts and organic base-formed salts (such as organic amines), such as benzathine, dicyclohexylamine, hypamine (salts formed with *N,N-*bis(dehydroabietyl)ethylenediamine), N-methyl-D-glucosamine, N-methyl-D-glucosamide, tert-butylamine, and salts formed by amino acids such as arginine, lysine and so on. An alkaline nitrogenous group can be combined with a halide to form a quaternary ammonium salt, such as with small molecular alkyl halide (such as chloride, bromide and iodide of methyl, ethyl, propyl or butyl), dialkyl sulfates (such as dimethyl sulfate, diethyl sulfate, dibutyl sulfate and dipentyl sulfate), long-chain halide (such as chloride, bromide and iodide of decyl, dodecyl, tetradecyl or tetradecyl), aralkyl halide (such as benzyl and phenyl bromide) and so on.

The prodrug and solvate of the compound according to the present invention are also within the scope. The term "prodrug" herein refers to a compound that, when used to treat related diseases, undergoes a chemical transformation via a metabolic or chemical process to produce the compound according to the present invention, salt, or solvate thereof. The compound according to the present invention includes solvate, such as hydrate. As used herein, the term "solvate" refers to a complex formed by the compound according to the present invention coordinating with a solvate molecule in a specific proportion. As used herein, the term "hydrate" refers to a complex formed by the compound according to the present invention coordinating with water, such as monohydrate.

The compound, salt or solvate according to the present invention may exist in tautomeric form (such as amide and imidoether). All of these tautomers are part of the present invention.

The stereoisomers of all compounds (such as those asymmetric carbon atoms that may exist due to various substitutions), including their enantiomeric and diastereoisomeric forms, fall within the scope of the present invention. The independent stereoisomer of the compound according to the present invention may not coexist with other isomers (such as in the form of a pure or substantially pure optical isomer with a special activity), or they may be a mixture, such as a racemate, or a mixture formed with all or part of other stereoisomers. The chiral center according to the present invention can be in either S or R configuration, as defined by the International Union of Pure and Applied Chemistry (IUPAC) in 1974. The racemic forms can be resolved by a physical method, such as fractional crystallization, or derivatization to enantiomers followed by separation via crystalization, or separation by chiral column chromatography. Individual optical isomer can be obtained from racemates by any suitable method, including but not limited to a traditional method, such as recrystallization after forming salt with an optically active acid.

The weight percentage of the compound according to the present invention obtained by preparation, separation and purification in turn is equal to or greater than 90%, e.g., equal to or greater than 95%, equal to or greater than 99% ("very pure" compound), as listed in the context of the description. The "very pure" compound according to the present invention as described herein is also a part of the present invention.

All configurational isomers of the compound according to the present invention are covered in the scope, including mixtures, pure or very pure forms. The definition of the compound according to the present invention includes both olefin isomers, cis (Z) and trans (E), as well as cis and trans isomers of carbocyclic ring and heterocyclic ring.

Throughout the specification, groups and substituents can be selected to provide a stable fragment or compound.

Some compounds according to the present invention may exist in specific geometric or stereoisomer forms. The present invention covers all compounds, including their cis and trans isomers, R and S enantiomers, diastereomers, (D) isomers, (L) isomers, racemic mixtures and other mixtures. In addition, asymmetric carbon atoms can represent substituents, such as alkyl. All isomers and mixtures thereof are encompassed in the present invention.

According to the present invention, the ratio of isomers in a mixture containing the isomers can vary. For example, the mixture with only two isomers can have the following combinations: 50:50, 60:40, 70:30, 80:20, 90:10, 95:5, 96:4, 97:3, 98:2, 99:1, or 100:0. All ratios of isomers are within the scope of the present invention. Similar ratios that can be easily understood by those skilled in the art, and ratios for mixtures of more complex isomers are also within the scope of the present invention.

The present invention also includes isotope-labeled compounds (also known as isotope compounds), equivalent to the original compounds disclosed herein. However, one or more atoms may normally be substituted by atoms that differ from their atomic weight or mass number. Examples of isotopes that can be used in the compound according to the present invention include H, carbon, nitrogen, oxygen, phosphorus, sulfur, fluorine and chlorine isotopes, such as ²H, ³H, ¹³C, ¹¹C, ¹⁴C, ¹⁵N, ¹⁸O, ¹⁷0, ³¹P, ³²P, ³⁵S, ¹⁸F and ³⁶Cl. The compounds, or enantiomers, diastereomers, isomers, or pharmaceutically acceptable salts or solvates thereof according to the present invention, in which isotopes described above or other isotopic atoms are contained in the compounds are within the scope of the present invention. Certain isotope-labeled compounds according to the present invention, such as the radioactive isotopes of ³H and ¹⁴C, are also within the scope, which are useful in tissue distribution experiments of medicaments and substrates. Tritium, i.e., ³H and carbon-14, i.e., ¹⁴C, can be relatively easily prepared and detected, so they are preferred among isotopes. In addition, heavier isotope substitution such as deuterium, i.e., ²H, has advantages in certain therapies due to its good metabolic stability, such as increasing half-lives in the body or reducing dosage, therefore be preferred in certain cases. Isotope-labeled compounds can be prepared in a general way by using the protocol disclosed in the examples and replacing the non-isotope reagents with a readily available isotope labeled reagents.

If the synthesis of a specific enantiomer of the compound according to the present invention is to be designed, it can be prepared asymmetrically, or by derivatizing with a chiral adjuvant, separating the resulting diastereomeric mixture, and then removing the chiral adjuvant to obtain a pure enantiomer. In addition, if the molecule contains a basic functional group, such as amino acid, or an acidic functional group, such as carboxyl group, diastereomeric salt can be formed with a suitable optically active acid or base, and then separated by a conventional means such as fractional crystallization or chromatography to provide a pure enantiomer.

As described herein, the compound according to the present invention can be combined with any number of substituents or functional groups to expand its scope. Generally, the term "substitute" appears either before or after the term "optional", and the general formula that includes substituents in the formula of the present invention, refers to substitution of a hydrogen radical with a substituent of a specified structure. When multiple positions in a particular structure are substituted by multiple specific substituents, the substituent at each position can be the same or different. The term "substitution" as used herein includes all permitted organic compound substitution. Broadly speaking, permissible substituents include non-cyclic, cyclic, branched, non-branched, carbocyclic ring and heterocyclic ring, aromatic ring and non-aromatic organic compound. In the present invention, heteroatom such as nitrogen can have hydrogen substituents or any permissible organic compound described above to supplement its valence state. Furthermore, the present invention is not intended to limit in any way permissible replacement of organic compounds. It is believed in the present invention that the combination of substituents and variable groups in the form of a stable compound is good for the treatment of diseases. The term "stable" herein refers to a compound that is stable and maintains the integrity of the compound structure in a period of time long enough to be detected, preferably be active in a period of time long enough for the purpose described herein above.

Metabolites of the compound and pharmaceutically acceptable salts thereof according to the present application, as well as prodrugs that can be transformed *in vivo* into a structure of the compound and pharmaceutically acceptable salts thereof according to the present application, are also included in the claims of the present application.

### Preparation method

The preparation methods of the compound with the structure shown in formula (I) according to the present invention are described more specifically below, while these specific methods are not intended to constitute any limitations to the present invention. The compound according to the present invention may also be optionally prepared by combining various synthetic methods described in the description or known in the art, such combinations may be easily carried out by those skilled in the art according to the present invention.

Generally, in the preparation process, each reaction is usually carried out under the protection of an inert gas, in an appropriate solvent, at room temperature to 90 °C, and the reaction time is usually 2-24 hours.

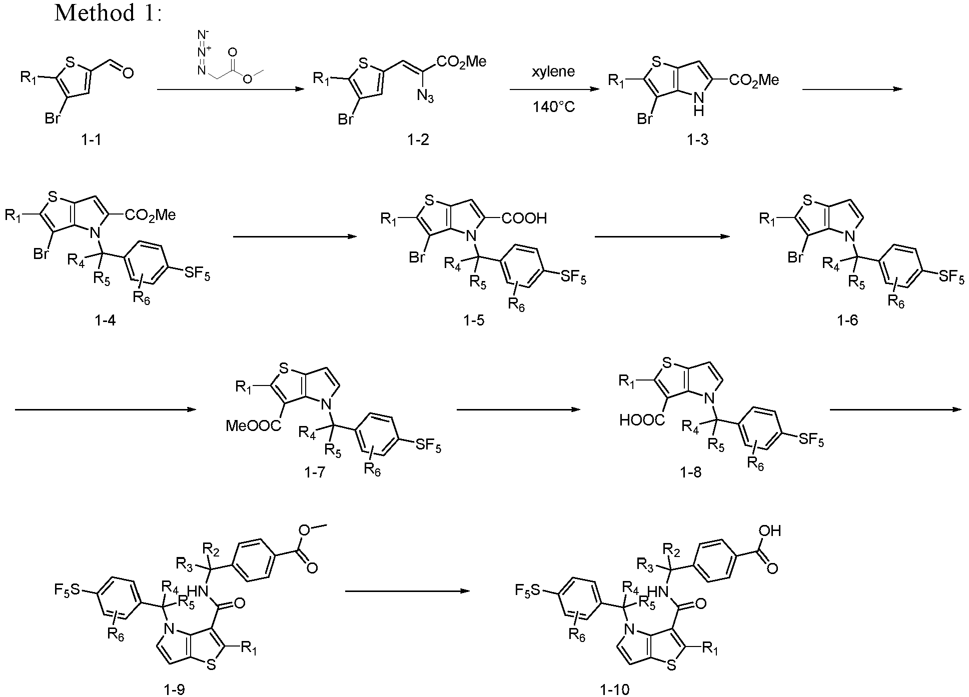

R₁, R₂, R₃, R₄, R₅, and R₆ in method 1 have the definitions described in the present invention. The method comprises the following steps:
(i) reacting methyl azidoacetate with the aldehyde group in compound 1-1 to give compound 1-2 in an organic solvent such as methanol; preferably, the reaction was carried out in the presence of a base, and more preferably, the base was NaOMe (sodium methoxide);
(ii) refluxing compound 1-2 in an organic solvent such as xylene to give compound 1-3;
(iii) reacting compound 1-3 with a benzyl bromide derivative to give compound 1-4 in an inert solvent (such as DMF) under alkaline conditions; preferably, the reaction was carried out in the presence of CsCO₃;
(iv) hydrolyzing methyl carboxylate in compound 1-4 to carboxylic acid to give compound 1-5 in an organic solvent (such as a mixed solvent of methanol and tetrahydrofuran) under alkaline conditions; preferably, the hydrolyzation was carried out in the presence of LiOH;
(v) reacting compound 1-5 with copper powder in quinoline under acidic conditions to give compound 1-6; preferably, the reaction was carried out in the presence of a catalyst (such as Cu);
(vi) catalyzing compound 1-6 by palladium to give compound 1-7 in an organic solvent (such as a mixed solvent of DMSO and methanol) under alkaline conditions in an atmosphere of carbon monoxide; preferably, the palladium catalyst used was PdCl₂dppf, and/or the temperature of the reaction was 75±5 °C;
(vii) hydrolyzing methyl carboxylate in compound 7 to carboxylic acid to give compound 1-8 in an organic solvent (such as a mixed solvent of methanol and tetrahydrofuran) under alkaline conditions; preferably, the hydrolyzation was carried out in the presence of LiOH;
(viii) condensing compound 1-8 with benzylamine to give compound 1-9 in an inert solvent (such as DMF); preferably, the condensation was carried out in the presence of HATU and DIEA; and
(ix) hydrolyzing methyl carboxylate in compound 1-9 to carboxylic acid to give compound 1-10 in an inert solvent (such as a mixed solvent of methanol and tetrahydrofuran) under alkaline conditions; preferably, the hydrolyzation was carried out in the presence of LiOH.

In the above reaction steps, the reaction solvent, reaction temperature, reaction time, catalyst, etc. can be selected according to the specific reactants.

### Pharmaceutical composition and administration method

Since the compounds according to the present invention are excellent antagonists of EP₄ receptors, the compounds according to the present invention and the various crystal forms, pharmaceutically acceptable inorganic or organic salts, hydrates, solvents, stereoisomers, isotope compounds or prodrugs thereof and pharmaceutical compositions described in the present invention can be used for the treatment or prevention of EP₄ receptor-related diseases such as prostaglandin E₂/EP₄ receptor-mediated diseases. The pharmaceutical composition described in the present invention can be used for the prevention and/or treatment of the following diseases: acute and chronic pain, inflammatory pain, diseases associated with inflammation, osteoarthritis and rheumatoid arthritis, and also used for the treatment of cancer.

The present invention provides a method for the treatment of inflammatory diseases sensitive to nonsteroidal anti-inflammatory drugs, comprising administering a non-toxic, therapeutically effective amount of the compound of formula I to a patient in need of such a treatment. In an embodiment comprising the above method, the patient is also at risk of a thrombotic cardiovascular event and/or gastrointestinal ulceration/bleeding.

Another embodiment of the present invention provides a method for the treatment of a prostaglandin E₂-mediated disorder that is favorably treated by an active agent that selectively antagonizes EP₄, rather than inhibiting COX-1/COX-2. The method comprises administering a non-toxic effective amount of the compound shown in Formula I to a patient in need of such a treatment. In an embodiment comprising the above method, the patient is also at risk of a thrombotic cardiovascular event.

By way of example, but not limited to, the compound described herein can be used for cancer immunotherapy targeting host immunosuppressive cells in the tumor microenvironment, which may be myeloid system or lymphatic system. In one embodiment, the compounds described herein can be used to treat patients with various tumor types, including those with high levels of myeloid infiltrates. The level of myeloid infiltrate can be determined, for example, based on The Cancer Genome Atlas (TCGA) or other sources. The type of such tumor can also be identified based on protein or gene (such as mRNA) expression analysis.

Types of tumors include, but not limited to, pancreatic adenocarcinoma, clear cell carcinoma of the kidney, squamous cell carcinoma of the head and neck (SCCHN), non-small cell lung cancer (NSCLC), colorectal cancer (CRC), hepatocellular carcinoma (HCC), serous epithelial ovarian cancer, cervical cancer, transitional cell bladder cancer, skin cancer, glioblastoma, kidney cancer, prostate cancer, pancreatic cancer and triple-negative breast cancer (TNBC).

The compound according to the present invention can be used to treat or prevent tumor formation in a subject in need of such a treatment or prevention. The treatment includes partial or complete suppression of tumor formation, spread, or metastasis, and partial or complete destruction of tumor cells. The term "prevention" includes complete prevention of the onset of clinically significant neoplasia or the prevention of the onset of clinically significant neoplasia in at-risk individuals. The definition is also intended to include preventing the origin of malignant cells or preventing or reversing the development of malignant procells to malignant cells. This includes preventive treatment for people at risk of developing neoplasm. The term "subject" for therapeutic purposes includes any human or mammalian subject with any known tumor, and preferably a human subject. For the prevention method, the subject is any human or animal subject, and preferably a human subject at risk of developing tumors. Subjects may be at risk due to exposure to carcinogens, genetically predisposed tumors, etc.

The compound of formula (I) can be combined with other drugs known to treat or improve similar pathologies. In combined administration, the mode and dose of the original drug can remain unchanged while the compound of formula I is taken simultaneously or subsequently. When the compound of formula I is taken simultaneously with one or more other drugs, it is preferable to use a pharmaceutical composition comprising one or several known drugs and the compound of formula I at the same time. Combined administration also involves taking compound of formula I with one or more other known drugs during overlapping time periods. When a compound of formula I is used in combination with one or more other drugs, the dose of the compound or the known drug may be lower than the dose thereof when used alone. Antitumor activity of EP₄ antagonists in various combinations with: radiation; antibodies against cytotoxic T lymphocyte antigen 4 (anti-CTLA4); antibodies against programmed death ligand 1 (anti-PDL1); antibodies against programmed cell death protein 1 (anti-PD1); and antimetabolites have been detected. The present invention further comprises a method for the treatment of cancer with an effective amount of the compound according to the present invention or using an effective amount of the compound according to the present invention in combination with an effective amount of the following substances: radiation; antibodies against cytotoxic T lymphocyte antigen 4 (anti-CTLA4); antibodies against programmed death ligand 1 (anti-PDL1); antibodies against programmed cell death protein 1 (anti-PD1); indoleamine-2,3-dioxygenase (IDO) inhibitors; tryptophan-2,3-dioxygenase (TDO) inhibitors; and antimetabolites. These antibodies can be selected from the group consisting of, but not limited to, MDX-010 (ipilimumab, Bristol-Myers Squibb), CP-675, 206 (tremelimumab, Pfizer), MPDL 3280 A (Roche), MDX-1106 (nivolumab, Bristol-MyersSquibb), labrolizumab (Merck) and pembrolizumab (Merck).

"Pharmaceutically acceptable carrier" refers to one or more compatible solid or liquid fillers or gel substances that are suitable for human use and that are of sufficient purity and sufficiently low toxicity. "Compatibility" herein refers to that the components of the composition can be blended with the compound according to the present invention without significantly reducing the efficacy of the compound. Partial examples of the pharmaceutically acceptable carrier are cellulose and derivatives thereof (such as sodium carboxymethylcellulose, sodium ethylcellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerin, mannitol, sorbitol, etc.), emulsifiers (such as Tween ^{®}), wetting agents (such as sodium lauryl sulfate), colorants, flavorings, stabilizers, antioxidants, preservatives, pyrogen-free water, etc.

"Pharmaceutically acceptable excipient" and "pharmaceutically acceptable carrier" refer to the substances that contribute to the formulation and/or administration of the active agent and/or absorption by an individual, and may be included in the composition of the present disclosure without causing a significant adverse toxicological effect on the individual. Non-limiting examples of pharmaceutically acceptable carriers and excipients include water, NaCl, saline solution, lactate Ringer's solution, conventional sucrose, conventional glucose, binders, fillers, disintegrants, lubricants, coatings, sweeteners, flavors, salt solutions (such as Ringer's solution), alcohols, oils, gelatin, carbohydrates, such as lactose, amylose or starch, fatty acid esters, hydroxymethyl cellulose, polyvinyl pyrrolidine and pigments and the like. Such a formulation can be sterilized and, if necessary, mixed with an auxiliary agent such as a lubricant, preservative, stabilizer, wetting agent, emulsifier, salt affecting osmotic pressure, buffer, colorant and/or aromatic substance that will not adversely react with the compound provided herein or interfere with the activity of the compound provided herein. Those skilled in the art will recognize that other drug carriers and excipients can also be suitable for use with the disclosed compounds.

In some embodiments, the pharmaceutical composition according to the present invention may be in solid or liquid form.

Drugs containing the active ingredient (i.e., compound shown in formula I) can be in suitable oral dosage forms, such as tablet, pill, lozenge, aqueous or oily suspension, dispersed latex powder or granule, emulsion, hard or soft capsule or syrup or elixir. Drugs for oral use can be prepared by any known process of the manufacturers of the pharmaceutical ingredients. These compositions may include one or more of the agents described below, such as sweetener, flavoring agent, colorant and protective agent, in order to provide an elegant and delicious pharmaceutical preparation. Tablets contain active ingredients mixed with non-toxic pharmaceutically acceptable excipients suitable for the production of tablets. Examples of these excipients include, inert diluent, such as calcium carbonate, sodium carbonate, lactose, calcium phosphate or sodium phosphate; granulation, disintegrant, such as cornstarch or alginic acid; binder, such as starch, gelatin or Arabic gum, and lubricant such as magnesium stearate, stearic acid or talc. The tablets can be uncoated, or coated to delay degradation and absorption in the gastrointestinal tract and thus maintain activity over a longer period of time.

The pharmaceutical composition according to the present invention can be administered parenterally, orally, buccally, sublingually, nasally, rectally, topically, or percutaneously. The pharmaceutical composition for oral administration is usually preferred.

The pharmaceutical composition according to the present invention suitable for oral administration will typically be discrete units in solid form, for example, in the form of tablet, capsule, cachet, powder, granule, lozenge, patch, suppository, pill, or in liquid form, such as liquid formulation, solution or suspension suitable for injection of infusion.

The precise amount of compound that delivers a therapeutically effective amount to an individual will depend on the mode of administration, the type and severity of the disease and/or condition, and the characteristics of the individual, such as general health, age, sex, weight, and tolerance to the drug. Those skilled in the art will be able to determine the appropriate dosage based on these and other factors. When administered in combination with other therapeutic agents, the "therapeutically effective amount" of any other therapeutic agent will depend on the type of drug used. Appropriate dosage for approved therapeutic agents is known, and can be adjusted by those skilled in the art according to the condition of the individual, the type of condition being treated and the amount of the compound according to the present invention used below, for example, as reported in the literature and recommended in the Physician's Desk Reference (57th edition, 2003).

In the pharmaceutical composition according to the present invention, the content of the active ingredient is typically 0.000001-1wt%, preferably 0.00001-1wt%, most preferably 0.0001-0.1wt%.

Examples of the subject to be administrated with a pharmaceutical composition or therapeutic agent according to the present invention include mammals (such as human, mouse, rat, hamster, rabbit, cat, dog, cow, sheep, monkey, etc.).

The present invention also provides a method for the preparation of the pharmaceutical composition, comprising the following steps: mixing the pharmaceutically acceptable carrier with the compound of general formula (I), pharmaceutically acceptable salt, hydrate or solvate thereof according to the present invention to form a pharmaceutical composition.

The present invention also provides a treatment method, comprising the following steps: administering to a subject in need the compound of general formula (I) or a crystal form, pharmaceutically acceptable salt, hydrate or solvate thereof according to the present invention, or the pharmaceutical composition according to the present invention, for selectively inhibiting a EP₄ receptor.

The present invention has at least one of the following advantages:
(1) the compound according to the present invention has an excellent inhibitory activity against a EP₄ receptor, and has a better selective inhibitory activity on a EP₄ receptor;
(2) the compound according to the present invention has lower toxic and side effects; and
(3) the compound according to the present invention has better pharmacodynamics, pharmacokinetic properties and druggability.

The present invention is further described below in conjunction with specific embodiments. It should be understood that these embodiments are intended only to illustrate the present invention and are not intended to limit the scope of the present invention. The following Examples, without indicating specific conditions of the experimental method, are generally performed in accordance with conventional conditions described by such as Sambrook et al., Molecular Cloning: Laboratory Manual (New York: Cold Spring Harbor Laboratory Press, 1989), or in accordance with the manufacturer's recommended conditions. Unless otherwise specified, percentages and parts are calculated by weight.

Unless otherwise defined, all professional and scientific terms used in the text have the same meanings as those familiar to those skilled in the art. In addition, any method and material similar or equal to the content described may be applied to the method of the present invention. The preferable embodiments and materials described herein are for demonstration purposes only.

The structure of the compound of the present invention is determined by nuclear magnetic resonance (NMR) and liquid chromatography-mass chromatography (LC-MS).

NMR was detected by using Bruker AVANCE-400 and Bruker AVANCE-500 NMR equipment, and the determination solvents included deuterated dimethyl sulfoxide (DMSO-d₆), deuterated acetone (CD₃COCD₃), deuterated chloroform (CDCl₃) and deuterated methanol (CD₃OD), etc.. The internal standard was tetramethylsilane (TMS), and the chemical shift was measured in parts per million (ppm).

Liquid chromatography-mass chromatography (LC-MS) was detected by using Agilent 1260 mass spectrometer. HPLC was determined by using Agilent 1100 high-pressure chromatograph (Microsorb 5 micron C18 100 × 3.0 mm column).

Qingdao GF254 silica gel plate was used for thin layer chromatography silica gel plate, a specification of 0.15-0.20 mm was used for TLC, and a specification of 0.4mm-0.5mm was used for preparation of thin layer chromatography. Column chromatography generally used Qingdao silica gel with 200-300 mesh silica gel as a carrier.

The starting materials in examples of the present invention are known and commercially available, or may be synthesized according to literature reported in the art.

Unless otherwise specified, all reactions of the present invention were carried out by continuous magnetic stirring under the protection of a dry inert gas (such as nitrogen or argon), and the reaction temperature was measured in degrees Celsius.

### The following abbreviations are used throughout the invention.

THF: tetrahydrofuran; MeOH: methanol; HCl: hydrochloric acid; Pd(PPh₃)₄: tetratriphenylphosphine palladium; K₂CO₃: potassium carbonate; AcOK: potassium acetate; NaOH: sodium hydroxide; H₂O: water; TEA: triethylamine; DIEA: N,N-diisopropylethylamine; DMF: N,N-dimethylformamide; DMA: N,N-dimethylacetamide; Py: pyridine; DCE: 1,2-Dichloroethane; DMSO: dimethyl sulfoxide; TFA: trifluoroacetic acid; NaBH(AcO)₃: triacetyl sodium borohydride; Sn₂(Bu-n)₆: hexabutylditin; AlCl₃: aluminum chloride; CuI: cuprous iodide; DPPA: diphenyl azide phosphate; BuOH: tert-butanol; Cs₂CO₃: cesium carbonate; K₃PO₄: potassium phosphate; BnBr: benzyl bromide; PD₂(DBA)₃: tris(dibenzylideneacetone)dipalladium; X-Phos: 2-dicyclohexylphosphine-2,4,6-triisopropylbiphenyl; EA: ethyl acetate; NaHCO₃: sodium bicarbonate; DIPEA: N,N-diisopropylethylamine; and HBr: hydrogen bromide.

### Examples

### Example 1. Synthesis of compound 1

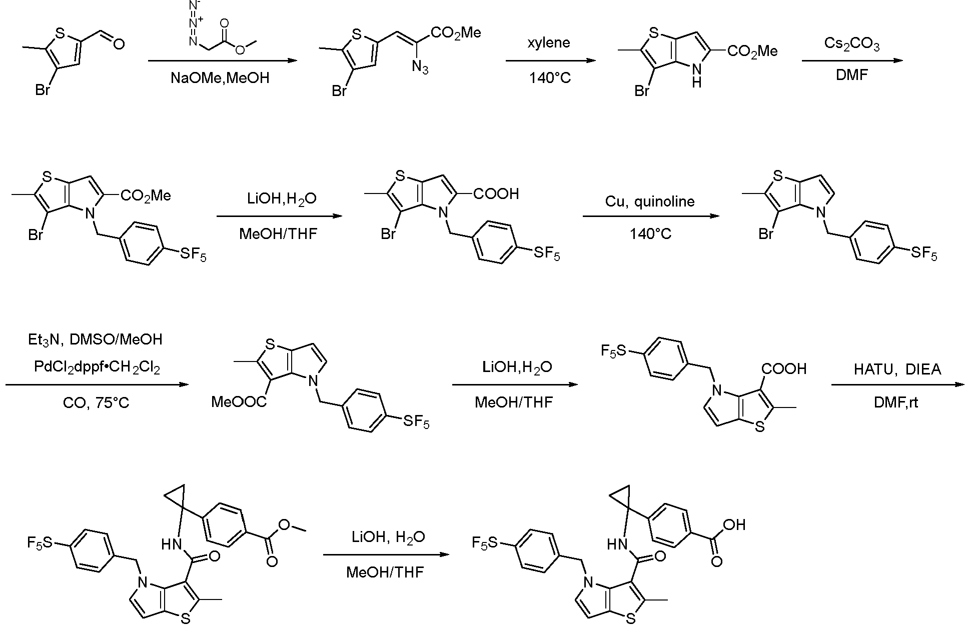

### Step 1: Synthesis of methyl 2-azido-3-(4-bromo-5-methylthien-2-yl)acrylate

Dissolving methyl azidoacetate (8.3 g, 72.8 mmol) and 4-bromo-5-methylthiophene-2-formaldehyde (5 g, 24 mmol) in 50mL of methanol, and adding a solution of NaOMe in methanol (13.5 mL, 5.4M) dropwise at -25°C. Stirring at 0 °C for 2 h and adding 50 mL of ice water. Stirring and filtering, and washing the filter cake with water and drying to give methyl 2-azido-3-(4-bromo-5-methylthiophen-2-yl)acrylate (3.6 g, 49%), which does not need to be purified and can be used in the next step.

### Step 2: Synthesis of methyl 3-bromo-2-methyl-4H-thieno[3,2-b]pyrrole-5-carboxylate

Adding dropwise the crude product (3.6 g) obtained in the previous step dissolved in xylene (50 mL) to the refluxed xylene (40 mL) within 10 minutes. After 20 minutes of reflux, cooling to room temperature, concentrating to remove the solvent to give a solid crude product. Dissolving the solid, extracting, drying and concentrating to give methyl 3-bromo-2-methyl-4*H*-thieno[3,2-b]pyrrole-5-carboxylate (3.2 g, 98%). MS: m/z (ESI): 273.95[M+H]⁺.

### Step 3: Synthesis of methyl 3-bromo-2-methyl-4-(4-(pentafluorothio)benzyl)-4H-thieno[3,2-b]pyrrole-5-carboxylate

Dissolving methyl 3-bromo-2-methyl-4*H*-thieno[3,2-*b*]pyrrole-5-carboxylate (2.5 g, 9.1 mmol), 4-(pentafluorothio)benzyl bromide (3.0g, 10.1 mmol) and cesium carbonate (9.0 g, 27.5 mmol) in DMF(20 mL), and stirring for 4 h at room temperature. After the reaction was complete, adding water to quench, separating the organic phase, washing with water, drying, and purifying by column chromatography (PE:EA=5:1) to give methyl 3-bromo-2-methyl-4-(4-(pentafluorothio)benzyl)-4*H*-thieno[3,2-*b*]pyrrole-5-carboxylate (3.0g, 90%) as a yellow solid. MS: m/z(ESI):489.95[M+H]⁺.

### Step 4: Synthesis of 3-bromo-2-methyl-4-(4-(pentafluorothio)benzyl)-4H-thieno[3,2-b]pyrrole-5-carboxylic acid

Dissolving methyl 3-bromo-2-methyl-4-(4-(pentafluorothio)benzyl)-4*H*-thieno[3,2-*b*]pyrrole-5-carboxylate (3.0 g, 6.1 mmol) in a solution of MeOH/THF with a ratio of 1:1 (40 mL) at room temperature, then adding H₂O(20 mL), lithium hydroxide (404 mg, 18.4 mmol), stirring at 55 °C for 2 h, concentrating to remove the organic solvent, adjusting the pH to 4-5 with citric acid aqueous solution, filtering the solid, washing and drying to give 3-bromo-2-methyl-4-(4-(pentafluorothio)benzyl)-4*H*-thieno[3,2-*b*]pyrrole-5-carboxylic acid (3.0g) as a yellow solid. MS: m/z(ESI):475.93[M+H]⁺.

### Step 5: Synthesis of 3-bromo-2-methyl-4-(4-(pentafluorothio)benzyl)-4H-thieno[3,2-b]pyrrole

Dissolving 3-bromo-2-methyl-4-(4-(pentafluorothio)benzyl)-4*H*-thieno[3,2-*b*]pyrrole-5-carboxylic acid (3.0g, 6.3 mmol) in 30 mL of quinoline, then adding 2.0 g of copper powder, reacting at 140°C for 16 h. Cooling to the room temperature, adding 6N HCl to acidify to acidity, extracting, washing, drying, and purifying by column chromatography (PE) to give 3-bromo-2-methyl-4-(4-(pentafluorothio)benzyl)-4*H-*thieno[3,2-b]pyrrole (1.21g, 44%) as a light yellow solid. MS m/z(ESI):431.94 [M+H]⁺.

### Step 6: Synthesis of methyl 2-methyl-4-(4-(pentafluorothio)benzyl)-4H-thieno[3,2-b]pyrrole-3-carboxylate

Dissolving 3-bromo-2-methyl-4-(4-(pentafluorothio)benzyl)-4*H*-thieno[3,2-b]pyrrole (0.7 g, 1.85 mmol), PdCl₂dppf.CH₂Cl₂ (680 mg, 0.83 mmol) and triethylamine (374 mg, 3.70 mmol) in 20 mL of dimethyl sulfoxide/methanol with a ratio of 1:1 at room temperature, heating overnight at 75 °C in CO (balloon pressure). After the reaction was complete, adding water to quench, extracting, washing, drying, and purifying by column chromatography (PE:EA=2:1) to give methyl 2-methyl-4-(4-(pentafluorothio)benzyl)-4*H-*thieno[3,2-*b*]pyrrole-3-carboxylate (0.4g, 52%) as a yellow solid. MS m/z(ESI): 412.04[M+H]⁺.

### Step 7: Synthesis of 2-methyl-4-(4-(pentafluorothio)benzyl)-4H-thieno[3,2-b]pyrrole-3-carboxylic acid

Dissolving methyl 2-methyl-4-(4-(pentafluorothio)benzyl)-4*H*-thieno[3,2-*b*]pyrrole-3-carboxylate (0.4 g, 0.97 mmol) in MeOH/THF with a ratio of 1:2 (15 mL) at room temperature, then adding H₂O (5 mL) and lithium hydroxide (116 mg, 0.85 mmol), stirring at 55 °C for 2 h, concentrating to remove the organic solvent, adjusting the pH to 4-5 with citric acid aqueous solution, filtering the solid, washing and drying to give 2-methyl-4-(4-(pentafluorothio)benzyl)-4*H*-thieno[3,2-*b*]pyrrole-3-carboxylic acid (340 mg, 85%) as a yellow solid. MS m/z(ESI):398.1[M+H]⁺.

### Step 8: Synthesis of methyl 4-(1-{[2-methyl-4-(4-(pentafluorothio)benzyl)-4H-thieno[3,2-b]pyrrole-3-carbonyl]amino}-cyclopropyl)benzoate

Dissolving methyl 2-methyl-4-(4-(pentafluorothio)benzyl)-4*H*-thieno[3,2-*b*]pyrrole-3-carboxylic acid (340 mg, 0.85 mmol), 4-(1-aminocyclopropyl)-benzoate (245 mg, 1.28 mmol), HATU (486 mg, 1.28 mmol) and DIEA (220 mg, 1.7 mmol) in DMF (10mL) at room temperature, stirring for 16 h at room temperature. After the reaction was complete, adding water to quench, extracting, washing, drying, and purifying by column chromatography (DCM:MeOH=20:1) to give methyl 4-(1-{[2-methyl-4-(4-(pentafluorothio)benzyl)-4*H*-thieno[3,2-*b*]pyrrole-3-carbonyl]amino}-cyclopropyl)benzoate (0.45 g, 92%) as a yellow solid. MS m/z(ESI):571.1[M+H]⁺.

### Step 9: Synthesis of 4-(1-{[2-methyl-4-(4-(pentafluorothio)benzyl)-4H-thieno[3,2-b]pyrrole-3-carbonyl]amino)-cyclopropyl)carboxylic acid

Dissolving methyl 4-(1-{[2-methyl-4-(4-(pentafluorothio)benzyl)-4*H*-thieno[3,2-*b*]pyrrole-3-carbonyl]amino}-cyclopropyl)benzoate (0.45 g, 0.79 mmol) in MeOH/THF with a ratio of 1:2 (30 mL) at room temperature, then adding H₂O (5 mL) and lithium hydroxide (190 mg,7.91 mmol), stirring at 55 °C for 2 h, concentrating to remove the organic solvent, adjusting the pH to 4-5 with citric acid aqueous solution, filtering the solid, washing and drying to give a crude product, beating the crude product with ether (10mL) to give 4-(1-{[2-methyl-4-(4-(pentafluorothio)benzyl)-4*H*-thieno[3,2-*b*]pyrrole-3-carbonyl]amino}-cyclopropyl)carboxylic acid **(compound 1)** (340 mg, 85%) as a yellow solid. MS m/z(ESI):557.1[M+H]⁺.

¹H NMR(400MHz):δ11.12(s, 1H), 8.98(s, 1H), 7.69-7.74 (m, 4H), 7.14 (d, 2H),7.06(s, 1H), 6.97 (d, 2H), 6.38(s, 2H), 5.43(s, 2H), 2.48(s, 3H), 1.17(m, 2H), 0.96 (m, 2H).

### Example 2

Other compounds in Table 1 are prepared in a manner similar to that in Example 1.

| Compound No. | Structure |
|---|---|
| **1** | 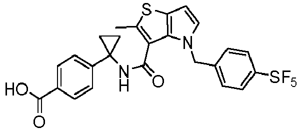 |
| **2** | 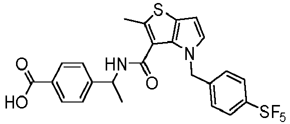 |
| **3** | 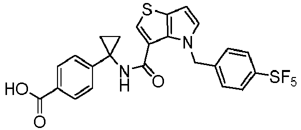 |
| **4** | 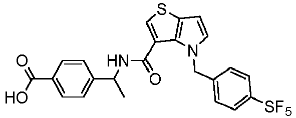 |

### Biological Activity Test Example 1:

### (a) Antagonist activity against EP4 receptor by cAMP assay:

Experiment Methods: Inoculating Flpin-CHO-EP₄ cells (8000 cells/well) into a 384-well plate (6007680-50, PE) with an assay buffer (1 × HBSS + 20 mM HEPES + 0.1% BSA + 500 µM IBMX). Preparing 8× compound working solution with the assay buffer. Adding 2.5 µL of the 8 × compound working solution to each well in the cell plate according to the plate map and incubating at 37 °C for 10 min. Preparing 8× PGE2 (400 nM) with the assay buffer. Adding 2.5 µL of the 8× PGE2 per well in the cell plate and incubating at 37 °C for 30 min. Diluting the Eu-cAMP tracer (1/50) with a lysis buffer and adding 10 µL per well in the test plate. Diluting Ulight-anti-cAMP (1/150) with a lysis buffer and adding 10 µL per well in the test plate. Incubating at room temperature for 1 h. Reading wavelength signal values at 665 nm and 615 nm on the Envision 2105 plate reader. The results are shown in Table 2.

**Table 2:**

| **Compound** | **Initial concentration (nM)** | **Dilution times (*)** | **IC₅₀ (nM)** |
|---|---|---|---|
| Control compound | 50 | 4 | 0.351 |
| Example 1 | 50 | 4 | 0.122 |

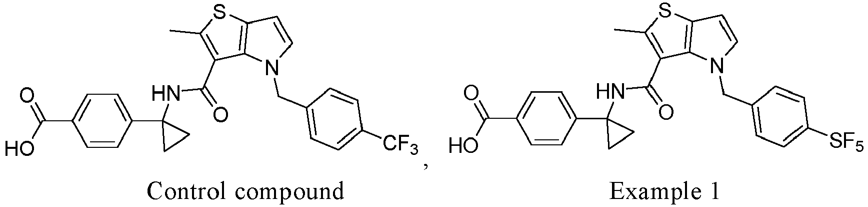

After testing, the compounds according to the present invention have excellent binding activity of EP₄ receptor, and have excellent inhibitory ability against EP₄ receptor.

### (b) Determination of PK of the compound according to the present invention:

Pharmacokinetics of compounds were studied in male Han Wistar rats. The compounds were administered intravenously as well as orally (n = 3 for each administration route). Sampling was performed at multiple time points after administration. Plasma extracts were quantitatively analyzed using a specific and sensitive LC-MS/MS bioanalytical method.

After testing, the compounds of the present invention have good pharmacodynamic and pharmacokinetic properties.

All documents referred to in the present invention are cited as references in the present application, just as each document is cited separately as a reference. Further, it should be understood that after reading the foregoing teachings of the present invention, those skilled in the art may make various changes or modifications to the present invention, and these equivalent forms also fall within the scope of the claims appended to the present application.

## Claims

1. A compound of formula I or a pharmaceutically acceptable salt, hydrate, solvate, stereoisomer, isotope compound or prodrug thereof; 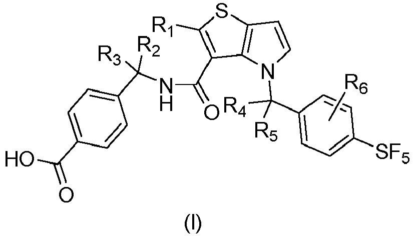 wherein,
R₁ is independently selected from the group consisting of: H, halogen, substituted or unsubstituted C₁₋₆ alkyl, and substituted or unsubstituted C₃₋₆ cycloalkyl;
R₂ and R₃ each is independently selected from the group consisting of: H, substituted or unsubstituted C₁₋₆ alkyl, and substituted or unsubstituted C₃₋₆ cycloalkyl; or, R₂ and R₃, together with the C atom to which they are attached, form a C₃₋₆ carbocyclic ring or a 3- to 6-membered heterocyclic ring containing one or two ring members each independently selected from the group consisting of S, O or NR_{b}; and the C₃₋₆ carbocyclic ring or 3- to 6-membered heterocyclic ring is optionally substituted with one or more Ri;
R_{b} each is independently selected from the group consisting of: H, substituted or unsubstituted C₁₋₆ alkyl, substituted or unsubstituted C₃₋₆ cycloalkyl, substituted or unsubstituted C₆₋₁₀ aryl, substituted or unsubstituted 5- to 6-membered heteroaryl, -C(O)R_{c}, and -S(O)₂R_{C};
R_{c} each is independently selected from the group consisting of: substituted or unsubstituted C₁₋₆ alkyl, substituted or unsubstituted C₃₋₆ carbocyclic ring, substituted or unsubstituted 3- to 6-membered heterocyclic ring, substituted or unsubstituted C₆₋₁₀ aryl, and substituted or unsubstituted 5- to 10-membered heteroaryl;
R₄ and R₅ each is independently selected from the group consisting of: H, and substituted or unsubstituted C₁₋₃ alkyl; or, R₄ and R₅, together with the C atom to which they are attached, form a substituted or unsubstituted C₃₋₆ carbocyclic ring; and
R₆ represents none or is selected from the group consisting of: halogen, CN, and haloalkyl;
unless otherwise specified, the substitution refers to that one or more of the hydrogens in a group is substituted by a substituent selected from the group consisting of: halogen, C₁₋₄ alkyl, and C₁₋₄ haloalkyl.

2. The compound of formula I or a pharmaceutically acceptable salt, hydrate, solvate, stereoisomer, isotope compound or prodrug thereof according to claim 1, wherein,
R₁ is independently selected from the group consisting of: H, halogen, C₁₋₆ alkyl, C₃₋₆ cycloalkyl, C₃₋₆ fluorocycloalkyl, and C₁₋₆ fluoroalkyl;
R₂ and R₃ each is independently selected from the group consisting of: H, C₁₋₆ alkyl, C₃₋₆ cycloalkyl, C₃₋₆ fluorocycloalkyl, C₁₋₆ fluoroalkyl; or, R₂ and R₃, together with the C atom to which they are attached, form a C₃₋₆ carbocyclic ring or a 3- to 6-membered heterocyclic ring; wherein, the heterocyclic ring contains one or two ring members each independently selected from the group consisting of S, O or NR_{b}; and the C₃₋₆ carbocyclic ring or 3- to 6-membered heterocyclic ring is further optionally substituted with R₁;
R_{b} is selected from the group consisting of: H, C₁₋₆ alkyl, C₃₋₆ cycloalkyl, C₃₋₆ fluorocycloalkyl, C₁₋₆ fluoroalkyl, C₆₋₁₀ aryl, 5- to 10-membered heteroaryl, C(O)-C₁₋₆ alkyl, C(O)-C₆₋₁₀ aryl, S(O)₂-C₁₋₆ alkyl, and S(O)₂-C₆₋₁₀ aryl;
R₄ and R₅ each is independently H or C₁₋₃ alkyl; and
R₆ represents none or a substituent selected from the group consisting of: halogen, CN, and trifluoromethyl.

3. The compound of formula I or a pharmaceutically acceptable salt, hydrate, solvate, stereoisomer, isotope compound or prodrug thereof according to claim 1 or 2, wherein, R₁ is selected from the group consisting of: H, halogen, C₁₋₆ alkyl, and C₃₋₆ cycloalkyl.

4. The compound of formula I or a pharmaceutically acceptable salt, hydrate, solvate, stereoisomer, isotope compound or prodrug thereof according to claim 1 or 2, wherein, R₂ and R₃ each is independently selected from the group consisting of H, and C₁₋₆ alkyl; or, R₂ and R₃, together with the C atom to which they are attached, form cyclopropyl.

5. The compound of formula I or a pharmaceutically acceptable salt, hydrate, solvate, stereoisomer, isotope compound or prodrug thereof according to claim 1 or 2, wherein, R₄ and Rs each is independently H or methyl.

6. The compound of formula I or a pharmaceutically acceptable salt, hydrate, solvate, stereoisomer, isotope compound or prodrug thereof according to claim 1 or 2, wherein, R₆ represents none or halogen.

7. The compound of formula I or a pharmaceutically acceptable salt, hydrate, solvate, stereoisomer, isotope compound or prodrug thereof according to claim 1 or 2, wherein, the compound is selected from the group consisting of: 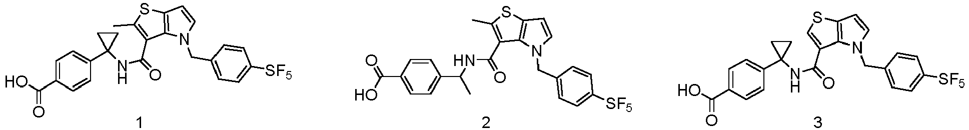 and

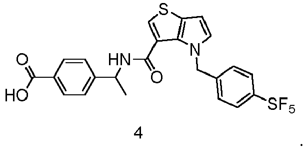

8. A pharmaceutical composition, comprising the compound of formula I or a pharmaceutically acceptable salt, hydrate, solvate, stereoisomer, isotope compound or prodrug thereof according to any one of claims 1 to 7, and a pharmaceutically acceptable carrier or diluent.

9. Use of the compound of formula I or a pharmaceutically acceptable salt, hydrate, solvate, stereoisomer, isotope compound or prodrug thereof according to any one of claims 1 to 7 or the pharmaceutical composition according to claim 8 in the preparation of (i) a medicament for the inhibition of EP₄ receptor and/or (ii) a medicament for the treatment or prevention of a disease associated with EP₄ receptor and/or (iii) a EP₄ receptor antagonist.

10. The use according to claim 9, wherein, the disease associated with EP₄ receptor comprises: acute and chronic pain, osteoarthritis, rheumatoid arthritis, cancer, or a combination thereof.
